(19) Europäisches Patentamt
European Patent Office
Office européen des brevets

(11) **EP 0 785 010 B1**

(12) **FASCICULE DE BREVET EUROPEEN**

(45) Date de publication et mention
de la délivrance du brevet:
**08.10.2003 Bulletin 2003/41**

(51) Int Cl.⁷: **B01D 17/05**, C07C 69/33

(21) Numéro de dépôt: **97400095.2**

(22) Date de dépôt: **17.01.1997**

(54) **Procédé de traitement d'un milieu aqueux pollué par des hydrocarbures et composition désémulsifiante et dispersante à base d'esters de polyglycérols**

Verfahren zur Behandlung eines mit Kohlenwasserstoff verunreinigten wässrigen Mediums, und entschäumende und zerstreuende Zusammensetzung auf der Basis von Polyglycerolestern

Method of treating an aqueous medium contaminated with hydrocarbons and defoaming and dispersant composition based on polyglycerol esters

(84) Etats contractants désignés:
**BE ES GB IT LU NL**

(30) Priorité: **22.01.1996 FR 9600671**

(43) Date de publication de la demande:
**23.07.1997 Bulletin 1997/30**

(73) Titulaire: **Institut Français du Pétrole**
**92852 Rueil-Malmaison Cedex (FR)**

(72) Inventeurs:
• **Dalmazzone, Christine**
**78000 Versailles (FR)**
• **Hillion, Gérard**
**95220 Herblay (FR)**

(56) Documents cités:
**DE-A- 3 346 097**     **GB-A- 1 557 182**
**US-A- 3 505 307**     **US-A- 4 321 146**

## Description

L'invention concerne un procédé de traitement d'une masse d'eau polluée par des composés hydrocarbonés, plus généralement par du pétrole. Elle concerne aussi une composition permettant notamment d'inhiber la formation d'une émulsion eau dans huile (pétrole, hydrocarbures) ou de la casser lorsqu'elle est déjà formée, la composition permettant enfin de favoriser à un domaine de concentrations appropriées la dispersion naturelle du pétrole dans l'eau.

Cette invention s'applique en particulier au traitement en mer des nappes de pétrole qui y ont été déversées.

L'art antérieur GB-A-1557.182 décrit une composition pour disperser des hydrocarbures provenant d'une pollution, contenant des esters d'acides carboxyliques du diéthylène glycol et du polyoxyéthylène glycol, éventuellement associés à un agent cotensioactif de type anionique. Ces esters dérivés du glycol n'ont rien à voir avec la composition selon l'invention et semblent devoir être utilisés dans des proportions importantes pouvant atteindre 10 %. L'arrière plan technologique est par ailleurs illustré par les brevets US-A-3505.307, US-A-4321.146 et DE-A-3346.097.

Le nettoyage ou la récupération de pétrole brut déversé en mer pose un grand nombre de problèmes. Pour lutter contre ce type de pollutions, il est possible de faire appel à des techniques physico-mécaniques permettant de collecter un film d'huile à la surface de l'eau. Il n'est cependant pas toujours facile d'utiliser ces techniques, surtout lorsque la surface de l'eau est trop agitée. De plus, ces moyens ne permettent que rarement une récupération totale de la pollution hydrocarbonée.

Dans beaucoup de situations réelles, on ne peut que laisser tout ou partie du polluant dans le milieu. Pour éviter que la nappe d'hydrocarbures ne se déplace jusqu'à la côte et la pollue, entraînée par des courants ou poussée par le vent, il est généralement recommandé d'utiliser la technique dite de dispersion qui consiste à assurer le fractionnement du film d'hydrocarbures en gouttelettes dispersées dans le volume d'eau jusqu'à une profondeur qui dépend de l'état d'agitation de la surface et des courants verticaux ou horizontaux stratifiés.

Cependant, la majeure partie des bruts déversés en mer forment rapidement des émulsions eau dans huile à cause de l'énergie résultant de l'action des vagues en surface et de la présence de surfactants et de particules tensio-actives naturellement présentes dans le pétrole à l'état brut. Ces composés, principalement asphaltènes et résines, et/ou complexes asphaltènes/cires, sont précipités sous forme de particules mouillées à la fois par la phase huile et la phase eau lorsque le pétrole brut s'altère et perd notamment par évaporation naturelle les aromatiques légers (benzène, toluène, xylène...) qui les maintenaient en solution. Ces tensio-actifs naturels agissent en formant un film viscoélastique à l'interface eau/huile très résistant, ce qui empêche la coalescence des gouttelettes d'eau de mer émulsifiées dans l'huile. Il se forme par conséquent très rapidement une émulsion eau dans huile très visqueuse, que l'on désigne généralement sous le nom de mousse au chocolat, qui peut contenir de 50 à 70 %, voire même jusqu'à 80 % d'eau en volume. Dès que l'émulsion commence à se former, il devient difficile de traiter la nappe avec des dispersants car le produit pénètre difficilement dans l'émulsion plus visqueuse que l'hydrocarbure initial. Avec certains pétroles, la période de temps pendant laquelle il est possible d'intervenir avec un traitement par dispersants est donc très réduite. De plus, le nettoyage et le stockage de telles émulsions posent de gros problèmes en mer, car le volume de l'huile initialement déversée peut facilement se trouver multiplié par 5.

Pour résoudre ce problème, on a développé depuis longtemps des désémulsifiants divers que l'on injecte dans l'émulsion eau dans pétrole afin de séparer l'eau de mer de l'hydrocarbure avant le stockage en mer (US Patent 4 316 806). Ces produits sont des surfactants qui agissent comme de puissants agents mouillants et ayant une importante activité interfaciale, capables de déplacer les tensio-actifs naturels qui stabilisent l'émulsion. Il y a quelques années, un autre concept est apparu en ce qui concerne le traitement de telles émulsions : pourquoi ne pas essayer d'empêcher la formation de ces émulsions in situ et favoriser leur dispersion dans le milieu naturel, voire les casser une fois formées ou lorsqu'elles commencent à se former, plutôt que de les traiter ensuite, ce qui nécessite des opérations de récupération et de traitement assez lourdes ? Il a donc été imaginé d'utiliser des produits inhibiteurs de formation d'émulsion.

Ce sont en fait des désémulsifiants qui doivent présenter des propriétés oléophiles, de façon à ne pas être lixiviés dans la phase aqueuse lors d'un traitement en mer, ce qui entraînerait rapidement une perte d'efficacité. Des études de laboratoire en Europe et au Canada ont montré que des traitements avec de faibles quantités de désémulsifiants selon l'art antérieur pouvaient inhiber la formation d'émulsions eau dans pétrole. La possibilité d'utiliser des désémulsifiants en application aérienne pour empêcher la formation d'émulsion et d'élargir ainsi les opportunités de traitement par dispersants est également connue. Il a également été constaté que des désémulsifiants ajoutés après la formation des émulsions pouvaient inverser le processus et casser l'émulsion, diminuant ainsi la viscosité de l'huile émulsifiée et autorisant une dispersion naturelle du pétrole. Suivant les circonstances, l'application aérienne de désémulsifiants sur une nappe de pétrole pourrait s'avérer une solution supplémentaire, voire alternative à l'utilisation des dispersants. Ces produits peuvent en effet être efficaces à des concentrations bien inférieures à celles requises dans le cas de dispersants (de l'ordre de 5 % pour les dispersants, inférieures à 1 % pour les inhibiteurs), ce qui représente un avantage significatif d'un point de vue pratique, logistique et financier en matière de traitement par application aérienne. A l'heure actuelle, les meilleurs produits commercialisés contiennent des copolymères oxyde d'éthylène/ oxyde de propylène tel que probablement le désémulsifiant Shell LA 1834® (Shell Chemicals UK) selon A. Lewis et M. Walker ("A Review of the Processes of Emulsification and Demulsification", in Proceedings of MSRC Emulsion Workshop, Technical Report Series 93-018, MSRC, Washington D.C., 1993, 223-238).

Malheureusement, ces produits ne sont pas biodégradables et sont de mauvais dispersants, à fortiori lorsqu'ils sont utilisés à faible concentration.

D'autres produits, généralement de HLB (balance hydrophile-lipophile) compris entre 3 et 6, sont de très bons désémulsifiants à faible concentration mais stabilisent les émulsions eau dans huile à des concentrations beaucoup plus élevées, de l'ordre du pourcent par rapport au pétrole ("Water in crude oil Emulsions from the Norwegian Continental Shelf.8. Surfactant and Macromolecular Destabilization" Urdahl O. et al., Colloids and surfaces. A: Physicochemical and Engineering Aspects, 74, 293-302 (1993)). C'est le cas du produit Triton N-42 de Berol-Nobel.

On peut de ce fait imaginer très facilement les problèmes que pourrait entraîner un dosage approximatif de la quantité de produit nécessaire pour casser une émulsion et qui aboutirait à l'effet inverse, c'est-à-dire à la formation d'une émulsion eau dans huile.

On a remarqué qu'en utilisant un produit comme les esters de polyglycérols d'origine naturelle, biodégradables et non toxiques, on obtenait d'excellents résultats. On a notamment constaté qu'en tant que désémulsifiant, il était capable d'empêcher la formation des mousses au chocolat et de casser les émulsions aussi bien aux faibles concentrations, par exemple inférieures à 400 ppm qu'aux fortes concentrations par exemple supérieures à 1 % par rapport au pétrole.

De plus, aux concentrations élevées, le produit selon l'invention peut favoriser la dispersion du pétrole dans l'eau, de préférence lorsqu'il est associé à un agent mouillant, que ce pétrole soit sous forme d'émulsion ou pas.

De manière plus détaillée, l'invention concerne un procédé de traitement d'un milieu aqueux pollué par des composés hydrocarbonés ou pétrole caractérisé en ce qu'on met en contact ledit pétrole avec une solution d'un mélange d'esters de polyglycérols dans une proportion pondérale d'au moins 50 ppm, telle qu'on inhibe la formation d'une émulsion eau dans pétrole, ou telle qu'on casse en partie l'émulsion si elle est déjà formée.

Selon une caractéristique avantageuse du procédé, on peut mettre en contact ledit pétrole avec la solution d'esters de polyglycérols dans une proportion pondérale de 120 à 50 000 ppm, avantageusement de 200 à 10 000 ppm et de préférence de 500 à 6 000 ppm pour inhiber ou pour casser l'émulsion.

Plus précisément, selon un premier mode de mise en oeuvre, on peut mettre en contact ledit pétrole avec la solution d'esters de polyglycérols dans une proportion pondérale de 1000 à 5 000 ppm telle qu'on casse l'émulsion et l'on récupère une phase hydrocarbonée par des moyens appropriés. Ces moyens sont en général mécaniques, par exemple un écrémeur et une pompe.

Selon un deuxième mode de mise en oeuvre, on peut mettre en contact le pétrole avec la solution d'esters de polyglycérols dans une proportion pondérale d'au moins 5 000 ppm telle que l'on casse l'émulsion et telle que l'on disperse une phase hydrocarbonée ainsi formée dans le milieu aqueux.

Par esters de polyglycérols, on entend un mélange de polyglycérols centrés sur une répartition di-tri-tétra glycérol, contenant de 5 à 20 % de glycérine libre et estérifié par au moins un acide gras saturé ou insaturé monocarboxylique ayant 6 à 24 atomes de carbone, et/ou ses esters correspondants (C1 à C4), ou par au moins un hydroxyacide aliphatique, comme l'acide ricinoléique par exemple, et/ou ses esters correspondants (C1 à C4), ou par une huile ou graisse (triglycéride) d'origine animale ou végétale ou par un mélange des produits précités.

Les esters de polyglycérols ont été décrits dans la littérature. Ils sont utilisés dans les secteurs de la cosmétique, de l'alimentaire ou de la pharmacie et sont pour cette raison fabriqués à partir de glycérine distillée commerciale.

Les polyglycérols ont en général la formule suivante :

$$CH_2 OH - CHOH - CH_2 (O CH_2 - CH - OH - CH_2)_{n-1} OH + (n-1) H_2O$$

avec 2 < n < 10

Selon le brevet FR-B-2677 643 incorporé comme référence, il est décrit l'utilisation, comme matière première de la synthèse de polyglycérols, soit de la glycérine brute encore basique issue d'une fabrication d'esters de corps gras à partir d'huiles, soit de la même phase alcaline glycérineuse après neutralisation par exemple par l'acide acétique ou par un acide carboxylique faible.

Selon la demande française de la Demanderesse FR 94/15834 incorporée comme référence, la matière première peut être de la glycérine neutralisée de préférence avec de l'acide sulfurique, chlorhydrique ou phosphorique.

L'estérification est également décrite dans ces brevets incorporés comme référence. Elle résulte de la réaction des polyglycérols ainsi formés avec un acide gras monocarboxylique aliphatique linéaire ayant 6 à 24 atomes de carbone par molécule, particulièrement à longue chaîne, ou avec un ester d'un tel acide et d'un monoalcool aliphatique ou avec une huile (glycéride) à une température par exemple de 170 à 250° C.

L'invention concerne aussi une composition particulièrement utile pour empêcher la formation d'une émulsion eau dans huile, ou casser l'émulsion au cas où elle serait formée ou en voie de se former puis pour favoriser la dispersion du pétrole dans le volume d'eau. Cette composition comprend en poids :

- de 0,1 à 80 % d'au moins un tensio-actif de préférence anionique,
- et de 20 à 99,9 % d'esters de polyglycérols selon l'invention.

Avantageusement, cette composition comporte en poids 5 à 50 % de composé tensio-actif et 50 à 95 % d'esters de polyglycérols.

Avantageusement, on peut utiliser comme composé anionique au moins un alkylsulfosuccinate d'un métal alcalin ou alcalino-terreux et de préférence du dioctylsulfosuccinate de sodium (appelé aussi di-(éthyl - 2 hexyl) sulfosuccinate de sodium), ou du dihexylsulfosuccinate de sodium, en solution par exemple dans un mélange d'eau et d'éthanol ou isopropanol.

Parmi les composés tensio-actifs non ioniques, on peut citer ceux qui sont biodégradables, en particulier les mélanges d'esters de sorbitol, éthoxylés ou non, par exemple un mélange de monooléate de sorbitol et de monooléate de sorbitol polyéthoxylé permettant d'avoir une valeur de HLB de 9 à 11 environ.

La composition selon l'invention est habituellement mise en solution dans au moins un solvant.

Le solvant entrant dans la composition des formulations est soit un monoalkyléther d'éthyléneglycol ou de polyéthyléneglycol (alkyl de $C_1$ à $C_6$), par exemple les monoéther éthylique, propylique, isopropylique et butylique de l'éthylèneglycol ou du diéthylèneglycol, soit un liquide hydrocarboné, par exemple une coupe pétrolière distillant entre 150 et 250°C ayant une teneur en hydrocarbures aromatiques inférieure à 5 % en poids, ou leur mélange.

La quantité de solvant généralement admise dans la composition n'excède pas 80 % en poids. Les modes d'application de la composition sont conventionnels. On peut par exemple pulvériser la composition dans le milieu aqueux marin pollué par des hydrocarbures soit manuellement à partir d'un réservoir sous pression, soit à partir d'un avion pour inhiber la formation d'une émulsion ou casser l'émulsion si elle est déjà formée.

En revanche, si l'on désire casser une émulsion récupérée il est préférable d'injecter en ligne la composition à l'entrée de la pompe de récupération.

Les exemples suivants illustrent l'invention.:

- Test d'inhibition et de cassage d'émulsion (méthode des éprouvettes en rotation)

Le test d'efficacité d'inhibition et de cassage d'émulsion est décrit dans la publication : Proceedings of the 18th AMOP, Environment Canada, Ottawa, Ontario, 317-327 (1995): IFP Procedure for Testing and Developing Water-in-Crude Oil Emulsion Inhibitors. (Dalmazzone et al.).

L'huile utilisé dans le cadre de ce test est un pétrole Arabian Light étêté à 150° C (BAL 150), qui présente l'avantage de former facilement des émulsions eau dans huile stables même sous faible agitation.

L'appareil est constitué d'un cadre en métal pouvant contenir six éprouvettes graduées de 100 ml pourvues à leur base d'un robinet en téflon pour permettre les renouvellements d'eau. Le cadre est entraîné par un moteur et permet aux éprouvettes de tourner sur elles-mêmes autour d'un axe horizontal. Cet appareil peut être utilisé pour la détermination des cinétiques de formation d'émulsion eau dans huile ainsi que pour estimer l'efficacité des inhibiteurs d'émulsion inverse et des désémulsifiants. Les expériences concernant les déterminations de cinétiques sont réalisées dans les conditions suivantes :

Rapport des volumes : eau de mer synthétique/huile/air = 10/1/7 et 5/1/7
Vitesse de rotation : 30 et 50 rpm
Temps de mélange : 30 minutes à heures
Température : 20° C

Après la période d'agitation, les déterminations de formation d'émulsion eau dans huile sont faites par observation des positions des interfaces huile/émulsion et émulsion/eau. Le pourcentage d'eau dans l'émulsion (% EM) est donné par la relation suivante :

$$\% \ EM = (h_{EM} - h_0)/h_{EM})100 \ \%$$

avec $h_{EM}$ et $h_0$: hauteur d'émulsion et d'huile respectivement.

Les expériences d'inhibition sont réalisées avec une vitesse de rotation de 50 rpm pendant 8 heures. La phase huile est prémélangée avec le tensio-actif à 500, 200, 100 ppm et 50 ppm. L'effet de dilution rencontré en mer est simulé par des changements réguliers d'eau de mer. Un screening rapide peut être fait en arrêtant la rotation toutes les 2 heures et en changeant la phase aqueuse après 15 minutes de repos. Le produit est considéré efficace lorsqu'il n'y a pas de formation d'émulsion.

On peut utiliser cet appareillage pour tester les désémulsifiants. On fabrique une émulsion stable contenant au moins 75 % en eau dans les éprouvettes en rotation. Le produit est ensuite déposé à la seringue de façon homogène sur le dessus de l'émulsion. On agite ensuite l'éprouvette à 50 rpm pendant 5 minutes. Le produit est considéré comme efficace s'il y a séparation totale des phases après arrêt de l'agitation.

- Test dispersant en dilution

Ce test dynamique pour estimer l'efficacité des dispersants est décrit dans la publication Oil and Chemical Pollution, 3 : 433-444 (1986/87) : Dispersant Effectiveness Evaluation in a Dynamic Flow-Through System : the IFP Dilution Test, (C. Bocard and G. Castaing). La dispersion se fait dans une cuve cylindrique de 5 litres, équipée d'une arrivée d'eau en dessous de la surface pour récupérer l'émulsion huile dans eau à la base de la cuve. L'énergie nécessaire est fournie par un anneau de métal qui bat périodiquement en dessous de la surface de l'eau. L'efficacité de dispersion est définie comme le pourcentage d'huile émulsionnée pendant une période de temps donnée par rapport à la quantité maximum d'huile récupérable dans le même temps dans des conditions théoriques de pseudo-solubilisation immédiate. Dans la procédure normalisée (NF.T.90 345), 4 g du fuel de référence (mélange fuel lourd/Arabian Light) sont déposés sur la surface de l'eau à l'intérieur d'un anneau de confinement. Le dispersant est ajouté goutte à goutte à l'aide d'une seringue de façon à être uniformément réparti sur l'huile. Le rapport dispersant/huile est de 5 % en poids.

Dans la procédure du test inhibiteur, l'efficacité des formulations inhibitrices est estimée pour des concentration de 1 et 0,5 % en poids de matières actives environ. Le surfactant ou mélange de surfactants est ajouté goutte à goutte ou prémélangé dans la phase huile.

Exemple 1

Préparation d'un ester d'acides gras de décaglycérol.

1 200 g de solution glycérineuse basique issue de la méthanolyse de l'huile de colza, dont la composition est la suivante : glycérol = 46,59 %, méthanol + eau = 46,05 %, acides gras = 6,4 % (sous la forme de savon de sodium), sodium total = 0,96 % (dont 0,52 % en savons et 0,44 % sous la forme d'alcoolate), sont chauffés dans un ballon agité pour éliminer par distillation le méthanol et l'eau. L'élimination totale de l'eau à pression atmosphérique n'est effective que lorsqu'on atteint 230-235° C. On recueille 553 g d'un mélange eau-méthanol. C'est à partir de 240°C que débute la réaction de déshydratation du glycérol. Le sodium présent dans la solution sous la forme de savon et d'alcoolate est le catalyseur de cette réaction.

Pour déterminer le taux de polycondensation désiré, par exemple ici, un décaglycérol, il suffit d'appliquer la formule suivante :

$$\frac{1\ 200 \times 46,59 \times (18 \times 9)}{100\ (92 \times 10)} = 98,45 \text{ g d'eau}$$

Au bout de 95 minutes, on recueille 98,40 cm$^3$ d'eau de réaction. La température finale de réaction est de 265° C. La composition du mélange de polyglycérols ainsi obtenu est la suivante : glycérine = 5,30 %, diglycérols = 20,35 %, triglycérols = 29,10 %, tétraglycérols = 13,50 %, pentaglycérols = 8,80 %, hexaglycérols = 7,10 %, heptaglycérols = 6,05 %, octaglycérols = 4,85 %, nonaglycérols = 3,50 %, décaglycérols = 1,45 %.

La transformation de ce mélange de polyglycérols en esters de polyglycérols est réalisée immédiatement à la suite après un refroidissement du mélange à 210-220° C, avec une stoechiométrie de 1,3 d'une coupe d'esters de méthyle d'acides gras en $C_8$-$C_{18}$ de poids moléculaire moyen de 227,5 et de composition : $C_8$ = 7,5 %, $C_{10}$ = 5,5 %, $C_{12}$ = 48,0 %, $C_{14}$ = 18,0 %, $C_{16}$ = 9,0 %, $C_{18}$ = 12,0 %.

218 g de la coupe d'esters d'acides gras sont ajoutés à raison de 6 g/min sur le mélange de polyglycérols à une température de 210-220°C. La transestérification est immédiate et du méthanol est distillé en continu jusqu'à concurrence de 30,73 g.

Après 1 heure de réaction on obtient 27 g de méthanol. On termine la réaction en opérant sous vide. Dans un premier temps, on opère sous un vide statique, puis dynamique, pour atteindre à 220° C une valeur de 10 à 15 mm de Hg. Le temps total de la réaction de transestérification est de l'ordre de 2 heures.

Le produit est refroidi et peut être utilisé en l'état ou dilué dans un solvant approprié.

On obtient 732 g d'esters de $C_8$-$C_{18}$ de décaglycérols. Les sels de sodium présents sont conservés dans le produit sous la forme de savons de sodium. Ils représentent 1,57 % poids en ion sodium.

Les sels de sodium contenus dans le produit de réaction proviennent du mélange statistique des acides en $C_8$-$C_{18}$ ayant servi à la synthèse de l'ester et des savons d'origine (ex. colza), contenus dans la glycérine de départ. Caractéristiques du produit fabriqué :

Indice d'acides : 2,38
Indice de saponification :93,55
Nombre d'eau :12,2
(méthode de Greenwald)
Efficacité :

Selon le test inhibiteur des éprouvettes en rotation, le produit est testé sur plusieurs types de pétroles qui forment facilement des émulsions eau dans huile : Arabian Light étêté à 150°C (BAL 150), Safanya et mélange Arabian Light/fuel lourd (du moins émulsifiable au plus émulsifiable). Il apparaît que dès l'injection de 50 ppm dans la phase huile, le produit de l'invention s'avère efficace en tant qu'inhibiteur d'émulsion. Il apparaît plus efficace quand il est utilisé à une concentration de 100 ppm.

En tant que casseur d'émulsion, le produit montre son efficacité, de préférence à partir de 1 000 ppm. Le test de dispersion montre qu'avec une quantité d'esters de polyglycérols de 10 000 ppm, on obtient d'excellents résultats.

Exemple 2

On reprend l'exemple 1 mais au lieu de tester l'efficacité en inhibition d'émulsion des esters de palmiste ($C_8$-$C_{18,}$ centrés sur $C_{12}$-$C_{14}$) de décaglycérols, on examine l'efficacité des esters de décaglycérols correspondant à l'acide 2 - éthylhexanoïque ($C_8$) et à l'acide oléique ($C_{18}$). On note respectivement une concentration de 200 ppm et de 100-ppm, nécessaire à l'obtention d'un même effet d'inhibition d'émulsion sur un même pétrole (Arabian Light étêté à 150° C). On note que l'efficacité a tendance à augmenter avec la longueur de la chaîne.

Exemple 3. Comparatif

On reprend l'exemple 1, mais au lieu d'utiliser des esters de palmiste de décaglycérols, on utilise le produit Shell LA 1834®. Ce produit s'avère efficace à partir de 200 ppm dans le test d'inhibition d'émulsion.

Exemple 4

On compare les propriétés d'inhibition et de dispersion relatives à la composition selon l'invention et à une composition selon l'art antérieur comprenant le désémulsifiant Shell LA 1834® associé au même tensio-actif, selon les méthodes de l'exemple 1. Les formulations A1, A2, A3, B1, B2, B3 ont les compositions suivantes en poids :

EPPG10 : esters de palmiste de décaglycérols
GEROPON DOS® (RHÔNE-POULENC): dioctysulfosuccinate de sodium en solution hydro-alcoolique (65 % matière active)
KETRUL 210® (TOTAL SOLVANTS): coupe Kérosène 210-240° C à faible teneur en hydrocarbures aromatiques (<5 %) en tant que solvant.

A1:  25 % EPPG10
     38 % GEROPON DOS® (dont 25 % matière active)
     37 % KETRUL 210®

A2 :  45 % EPPG10
      8 % GEROPON DOS® (dont 5 % matière active)
      47 % KETRUL 210®

A3 :  38 % EPPG10
      18 % GEROPON DOS® (dont 12 % matière active)
      44 % KETRUL 210®

B1 :  25 % SHELL LA1834®
      38 % GEROPON DOS®
      37 % KETRUL 210®

B2 :   45 % SHELL LA1834®
        8 % GEROPON DOS®
        47 % KETRUL 210®

B3 :   38 % SHELL LA 1834®
        18 % GEROPON DOS®
        44 % KETRUL 210®

4-1 Le pétrole sur lequel ont porté les tests est le BAL 150 de l'exemple 1. Toutes ces formulations sont inhibitrices à 200 ppm de matières actives par rapport à l'huile (test inhibiteur).

4-2 On peut comparer leur action en dispersion sur le mélange Arabian light/fuel lourd de l'exemple 1 (test dynamique en dilution) à une concentration de 2 % (soit 1 % en matières actives), concentration préconisée pour avoir une action dispersante observable avec le test en dilution. Les résultats sont donnés dans le tableau ci-après :

| Formulation | Efficacité (%) |
|---|---|
| A1 | 48 |
| B1 | 32 |
| A2 | 31 |
| B2 | 25 |
| A3 | 29 |
| B3 | 13 |
| Geropon DOS® + Ketrul 210® | 12 |

On met en évidence que les formulations selon l'invention sont les plus efficaces en dispersion.

4-3 On a également regardé le cas de l'action en dispersion sur un pétrole plus léger : BAL 150, toujours à 2 % de produit par rapport à l'huile (soit 1 % en matière active).

| Formulation | Efficacité (%) |
|---|---|
| A3 | 36 |
| B3 | 16 |
| Geropon DOS® + Ketrul 210® | 10 |

Dans ce cas également, le produit selon l'invention est plus efficace.

Exemple 5

On a comparé les esters de polyglycérols selon l'invention avec des esters de polyglycérols distribués en France, qui sont des produits préparés à partir de glycérine distillée.

TRIODAN 55 (GRINSTED) ® : esters de polyglycérols (di, tri, tétra)
ISOLAN GO33® et GI34® (GOLDSCHIMDT FRANCE SA) : respectivement esters oléique et stéarique de polyglycérols
EMCOL 14® (WITCO) : oléate de polyglycérols
ADMUL PGE 1405 et 1410® (QUEST INTERNATIONAL) : esters de polyglycérols (di, tri, tétra)
PLUROL Stéarique WL 1009 (Gattefossé) : palmitostéarate de polyglycérols

Les seuls produits efficaces en inhibiteurs à 200 ppm sont : ADMUL PGE 1410 (C) ® PLUROL Stéarique WL 1009 (D)® et TRIODAN 55 (E) ®.

Chacun de ces produits a été formulé avec du GEROPON DOS® et du solvant KETRUL 210® (formulations C1, D1 et E1 dans des proportions identiques à la formulation A1 de l'exemple 4). Ils ont ensuite été testés avec le test dynamique en dilution à 1 et 5 % par rapport au mélange Arabian Light/fuel lourd.

| Produit | Efficacité (1 %) | Efficacité (5 %) |
|---|---|---|
| A1 | 37 | 54 |

(suite)

| Produit | Efficacité (1 %) | Efficacité (5 %) |
|---------|------------------|------------------|
| C1 | 14 | 40 |
| D1 | <10 | <10 |
| E1 | <10 | <10 |

Seul le produit C1, parmi les produits du commerce, présente une action dispersante. Ses performances sont cependant très inférieures celles de la composition obtenue à partir de glycérine brute.

**Revendications**

1.  Procédé de traitement d'un milieu aqueux pollué par des composés hydrocarbonés ou pétrole **caractérisé en ce qu'**on met en contact ledit pétrole avec une solution d'un mélange d'esters consistant essentiellement en des esters de polyglycérols, les polyglycérols ayant la formule suivante $CH_2 OH - CHOH - CH2 (O CH_2 - CH - OH - CH_2)_{n-1} OH$ avec $2 < n < 10$, dans une proportion pondérale d'au moins 50 ppm, telle que l'on inhibe la formation d'une émulsion eau dans pétrole ou telle que l'on casse au moins en partie l'émulsion si elle est déjà formée.

2.  Procédé selon la revendication 1, dans lequel on met en contact ledit pétrole avec la solution d'esters de polyglycérols dans une proportion pondérale de 200 à 10 000 ppm et de préférence de 500 à 6 000 ppm.

3.  Procédé selon la revendication 1 dans lequel on met en contact ledit pétrole avec la solution d'esters de polyglycérols dans une proportion pondérale de 1 000 à 5 000 ppm, telle que l'on casse l'émulsion et l'on récupère une phase hydrocarbonée par des moyens appropriés.

4.  Procédé selon la revendication 1 dans lequel on met en contact ledit pétrole avec la solution d'esters de polyglycérols dans une proportion pondérale d'au moins 5 000 ppm, telle que l'on casse l'émulsion et telle que l'on disperse une phase hydrocarbonée ainsi formée dans le milieu aqueux.

5.  Procédé selon l'une des revendications 1 à 4 dans lequel la solution comprend au moins un tensio-actif et de préférence au moins un tensio-actif anionique, dans les proportions pondérales suivantes :

    -   20 à 99,9 % d'esters de polyglycérols, de préférence 50 à 95 %
    -   0,1 à 80 % de composé tensio-actif, de préférence 5 à 50 %

    le tout dans au moins un solvant présent dans la composition finale dans une proportion n'excédant pas 80% en poids de ladite composition.

6.  Procédé selon la revendication 5 dans lequel les esters de polyglycérols résultent de la réaction des polyglycérols avec au moins un acide gras saturé ou insaturé monocarboxylique ayant 6 à 24 atomes de carbone, et/ou ses esters correspondants (C1 à C4), ou par au moins un hydroxyacide aliphatique comme l'acide ricinoléique par exemple et/ou ses esters correspondants (C1 à C4), ou par une huile ou graisse (triglycéride) d'origine animale ou végétale, ou par un mélange des produits précités.

7.  Procédé selon l'une des revendications 5 à 6 dans lequel le tensio-actif est au moins un alkylsulfosuccinate d'un métal alcalin ou alcalino-terreux.

8.  Procédé selon l'une des revendications 5 à 7 dans lequel le tensio-actif est du dioctylsulfosuccinate de sodium.

9.  Procédé selon l'une des revendications 1 à 8 dans lequel les esters de polyglycérols sont préparés à partir de glycérine brute (non distillée).

10. Composition ayant des propriétés désémulsifiantes et dispersantes dans un milieu aqueux comprenant de l'eau et des composés hydrocarbonés, ou du pétrole, comportant en poids, en présence d'au moins un solvant :

    -   de 0,1 à 80 % d'au moins un composé tensio-actif de préférence anionique

- et de 20 à 99,9 % d'esters de polyglycérols.

11. Composition selon la revendication 10, comportant en poids de 5 à 50 % de composé tensio-actif et 50 à 95 % d'esters de polyglycérols.

12. Composition selon les revendications 10 et 11 dans laquelle les esters de polyglycérols résultent de la réaction des polyglycérols avec au moins un acide gras saturé ou insaturé monocarboxylique ayant 6 à 24 atomes de carbone, et/ou ses esters correspondants (C1 à C4), ou par au moins un hydroxyacide aliphatique comme l'acide ricinoléique par exemple et/ou ses esters correspondants (C1 à C4), ou par une huile ou graisse (triglycéride) d'origine animale ou végétale ou par un mélange des produits précités, et dans laquelle le composé tensio-actif est au moins un alkysulfosuccinate d'un métal alcalin ou alcalino-terreux.

13. Composition selon la revendication 12 dans laquelle le composé tensio-actif est du dioctylsulfosuccinate de sodium.

14. Composition selon la revendication 12 ou 13 dans laquelle les polyglycérols sont préparés à partir de glycérine brute.

**Patentansprüche**

1. Verfahren zur Behandlung eines wässrigen Milieus, das durch kohlenwasserstoffhaltige Verbindungen oder Erdöl verschmutzt ist, **dadurch gekennzeichnet, dass** man das Erdöl mit einer Lösung von einem Estergemisch kontaktiert, das im Wesentlichen aus Polyglycerinestern besteht, wobei die Polyglycerine die folgende Formel haben $CH_2 OH - CHOH - CH_2 (O CH_2- CH - OH - CH_2)_{n-1} OH$ mit $2 < n < 10$, in einem Gewichtsanteil von wenigstens 50 ppm, derart, dass man die Bildung einer Emulsion Wasser in Erdöl behindert oder derart, dass man wenigstens teilweise die Emulsion zerkleinert, wenn sie bereits gebildet ist.

2. Verfahren nach Anspruch 1, bei dem man das Erdöl mit der Lösung von Polyglycerinestern in einem Gewichtsanteil von 200 bis 10.000 ppm und vorzugsweise von 500 bis 6.000 ppm kontaktiert.

3. Verfahren nach Anspruch 1, bei dem man das Erdöl mit der Polyglycerinesterlösung in einem Gewichtsanteil von 1.000 bis 5.000 ppm derart kontaktiert, dass man die Emulsion verkleinert, und man eine kohlenwasserstoffhaltige Phase durch geeignete Mittel gewinnt.

4. Verfahren nach Anspruch 1, bei dem man das Erdöl mit der Lösung von Polyglycerinestern in einem Gewichtsanteil von wenigstens 5.000 ppm derart kontaktiert, dass man die Emulsion zerkleinert, und derart, dass man eine so gebildete Kohlenwasserstoffphase in dem wässrigen Milieu dispergiert.

5. Verfahren nach einem der Ansprüche 1 bis 4, bei dem die Lösung wenigstens ein tensioaktives Mittel und vorzugsweise wenigstens ein anionisches Tensioaktivmittel in den folgenden Gewichtsanteilen umfasst:

   - 20 bis 99,9 % Polyglycerinester, vorzugsweise 50 bis 95 %
   - 0,1 bis 80 % einer tensioaktiven Verbindung, vorzugsweise 5 bis 50 %

   das Ganze in wenigstens einem Lösungsmittel, das in der Endzusammensetzung in einem Anteil vorliegt, der 80 Gew.-% dieser Zusammensetzung nicht überschreitet.

6. Verfahren nach Anspruch 5, bei dem die Polyglycerinester auf der Reaktion der Polyglycerine mit wenigstens einer gesättigten oder ungesättigten Monocarboxylfettsäure mit 6 bis 24 Kohlenstoffatomen resultiert, und/oder deren entsprechenden Estern (C1 bis C4) oder durch wenigstens eine aliphatische Hydroxysäure wie zum Beispiel Rizinusölsäure und/oder deren entsprechende Ester (C1 bis C4) oder durch ein Öl oder Fett (Triglycerid) tierischen oder pflanzlichen Ursprungs oder durch ein Gemisch der vorgenannten Produkte.

7. Verfahren nach einem der Ansprüche 5 bis 6, bei dem das tensioaktive Mittel wenigstens ein Alkylsulfosuccinat eines Alkali- oder Erdalkalimetalls ist.

8. Verfahren nach einem der Ansprüche 5 bis 7, bei dem das tensioaktive Mittel Natriumdioctylsulfosuccinat ist.

**9.** Verfahren nach einem der Ansprüche 1 bis 8, bei dem die Polyglycerinester ausgehend von (nicht destilliertem) Rohglycerin hergestellt werden.

**10.** Zusammensetzung mit den entemulgierenden und dispergierenden Eigenschaften in einem wässrigen Milieu, umfassend Wasser und Kohlenwasserstoffverbindungen oder Erdöl, umfassend an Gewicht in Gegenwart wenigstens eines Lösungsmittels:

- 0,1 bis 80 % wenigstens einer tensioaktiven, vorzugsweise anionischen Verbindung
- und 20 bis 99,9 % Polyglycerinester.

**11.** Zusammensetzung nach Anspruch 10, an Gewicht umfassend 5 bis 50 % tensioaktiver Verbindung und 50 bis 95 % Polyglycerinester.

**12.** Zusammensetzung nach den Ansprüchen 10 und 11, in der die Polyglycerinester aus der Reaktion der Polyglycerine mit wenigstens einer gesättigten oder ungesättigten Monocarboxylfettsäure mit 6 bis 24 Kohlenstoffatomen und/oder deren entsprechenden Estern (C1 bis C4) oder durch eine aliphatische Hydroxysäure wie beispielsweise Rizinusölsäure und/oder deren entsprechende Ester (C1 bis C4) oder durch ein Öl oder Fett (Triglycerid) tierischen oder pflanzlichen Ursprungs oder durch ein Gemisch der vorgenannten Produkte resultiert und bei der die tensioaktive Verbindung wenigstens ein Alkylsulfosuccinat eines Alkali- oder Erdalkalimetalls ist.

**13.** Zusammensetzung nach Anspruch 12, bei der die tensioaktive Verbindung ein Natrium-Dioctylsulfosuccinat ist.

**14.** Zusammensetzung nach Anspruch 12 oder 13, bei der die Polyglycerine ausgehend von Rohglycerin hergestellt werden.

## Claims

**1.** A process for the treatment of an aqueous medium which has been polluted with hydrocarbon compounds or crude oil, **characterized in that** said crude oil is brought into contact with a solution of a mixture of esters consisting erssentially of polyglycerol esters, the polyglycerols having the following formula $CH_2OH\text{-}CHOH\text{-}CH_2$ $(OCH_2\text{-}CH\text{-}OH\text{-}CH_2)_{n-1}OH$, where $2 < n < 10$, in a proportion of at least 50 ppm by weight in order to inhibit formation of a water-in-crude oil emulsion or to partially break the emulsion if it has already formed.

**2.** A process according to claim 1, in which said crude oil is brought into contact with the solution of polyglycerol esters in a proportion of 200 to 10000 ppm by weight, preferably 500 to 6000 ppm by weight.

**3.** A process according to claim 1, in which said crude oil is brought into contact with the solution of polyglycerol esters in a proportion of 1000 to 5000 ppm by weight to break the emulsion and recover a hydrocarbon phase using suitable means.

**4.** A process according to claim 1, in which said crude oil is brought into contact with the solution of polyglycerol esters in a proportion of at least 5000 ppm by weight to break the emulsion and disperse the hydrocarbon phase formed in the aqueous medium.

**5.** A process according to any one of claims 1 to 4, in which the solution comprises at least one surfactant, preferably at least one anionic surfactant, in the following proportions by weight:

- 20% to 99.9% of polyglycerol esters, preferably 50% to 95%;
- 0.1% to 80% of surfactant, preferably 5% to 50%,

the whole being in at least one solvent present in the filnal composition in a proportion not exceeding 80% by weight of said composition.

**6.** A process according to claim 5, in which the polyglycerol esters result from the reaction of polyglycerols with at least one saturated or unsaturated monocarboxylic fatty acid containing 6 to 24 carbon atoms, and/or its corresponding esters ($C_1$ to $C_4$), or by at least one aliphatic hydroxyacid such as ricinoleic acid and/or its corresponding esters ($C_1$ to $C_4$), or by an oil or grease (triglyceride) of animal or vegetable origin, or by a mixture of the above products.

7.  A process according to claim 5 or claim 6, in which the surfactant is at least one alkylsulphosuccinate of an alkali or alkaline-earth metal.

8.  A process according to any one of claims 5 to 7, in which the surfactant is sodium dioctylsulphosuccinate.

9.  A process according to any one of claims 1 to 8, in which the polyglycerol esters are prepared from unrefined (undistilled) glycerine.

10.  A composition with demulsifying and dispersing properties in an aqueous medium comprising water and hydrocarbon compounds or crude oil, comprising by weight, in the presence of at least one solvent:

    *   0.1% to 80% of at least one surfactant, preferably anionic; and
    *   20% to 99.9% of polyglycerol esters.

11.  A composition according to claim 10, comprising 5% to 50% by weight of surfactant and 50% to 95% of polyglycerol esters.

12.  A composition according to claim 10 and claim 11, in which the polyglycerol esters result from the reaction of polyglycerols with at least one saturated or unsaturated monocarboxylic fatty acid containing 6 to 24 carbon atoms, and/or its corresponding esters ($C_1$ to $C_4$), or by at least one aliphatic hydroxyacid such as ricinoleic acid and/or its corresponding esters ($C_1$ to $C_4$), or by an oil or grease (triglyceride) of animal or vegetable origin, or by a mixture of the above products, and in which the surfactant is at least one alkylsulphosuccinate of an alkali or alkaline-earth metal.

13.  A composition according to claim 12, in which the surfactant is sodium dioctylsulphosuccinate.

14.  A process according to claim 12 or claim 13, in which the polyglycerols are prepared from unrefined glycerine.